# EUROPÄISCHE PATENTSCHRIFT

(11) **EP 2 082 687 B1**
(45) Veröffentlichungstag und Bekanntmachung des Hinweises auf die Patenterteilung: **01.04.2015**
(21) Anmeldenummer: 08164757.0
(22) Anmeldetag: 22.09.2008
(51) Int. Cl.: A61B 6/00

(54) **Überlagerte Darstellung von Aufnahmen**
Overlaid presentation of exposures
Représentation superposée de saisies

(30) Priorität: 22.01.2008 EP 08150480
(43) Veröffentlichungstag der Anmeldung: 29.07.2009
(73) Patentinhaber: Brainlab AG, 85622 Feldkirchen (DE)
(72) Erfinder: Uhde, Jörg, 80538, München (DE); Bogojevic, Aleksander, 80636 München (DE)
(74) Vertreter: Schwabe - Sandmair - Marx

(56) Entgegenhaltungen:
- EP-A- 1 782 734
- DE-A1- 10 015 815
- DE-A1- 10 210 645
- DE-A1- 10 215 808
- DE-A1- 10 322 739
- DE-A1- 10 360 025
- GB-A- 2 433 668
- US-A1- 2003 179 856
- US-A1- 2006 115 054
- US-A1- 2007 003 016

## Beschreibung

Die Erfindung betrifft ein Verfahren zur orientierten, überlagerten Darstellung von bevorzugt überlappenden Aufnahmen von einem in wenigstens zwei Aufnahmeschritten mit einem Navigationssystem aufgenommenen, abzubildenden Bereich, auf wenigstens einem Bildschirm, gemäß dem Anspruch 1.

Insbesondere wird ein Verfahren vorgeschlagen, um von einem Körperbereich eines Patienten mittels Röntgen erstellte Aufnahmen zum Zwecke der computergestützten Navigation orientiert zueinander überlappend insbesondere mit maximierter Bildinformation darzustellen.

Um aussagekräftige Bilder im Hinblick auf Körperteile eines Menschen oder Tieres beispielsweise für die Orthopädie oder die Traumatologie zu erhalten, ist es üblich, mit einem Röntgengerät beispielsweise zwei Aufnahmen eines Körperbereiches, d.h., etwa einer anatomischen Struktur, zu machen.

Die wenigstens zwei Röntgenaufnahmen werden durch eine örtliche Verlagerung des Röntgengerätes relativ zu dem aufzunehmenden Bereich des Patienten, erhalten.

Üblicherweise werden dabei mehrere Aufnahmen auf zwei getrennten Monitoren abgebildet. Eine ganz übliche Konfiguration ist ein mobiles Röntgengerät, auch als "C-Bogen" bezeichnet, in Verbindung mit zwei Monitoren. Dabei wird auf einem Monitor ein aktuelles und auf einem weiteren Moitor ein beliebiges vorher aufgenommenes Bild des interessierenden Körperbereichs angezeigt, welches jederzeit durch das aktuelle Bild ersetz werden kann. Problematisch ist hierbei, dass die Bilder keine definierte Orientierung, keine maßstäbliche Beziehung und dergleichen, zueinander haben. D.h., die dargestellten Bilder können zueinander in sämtlichen Raumrichtungen verkippt sein, und der Anschluss der Bilder aneinander sowie die Darstellung mit dem gleichen Maßstab sind systematisch nicht möglich.

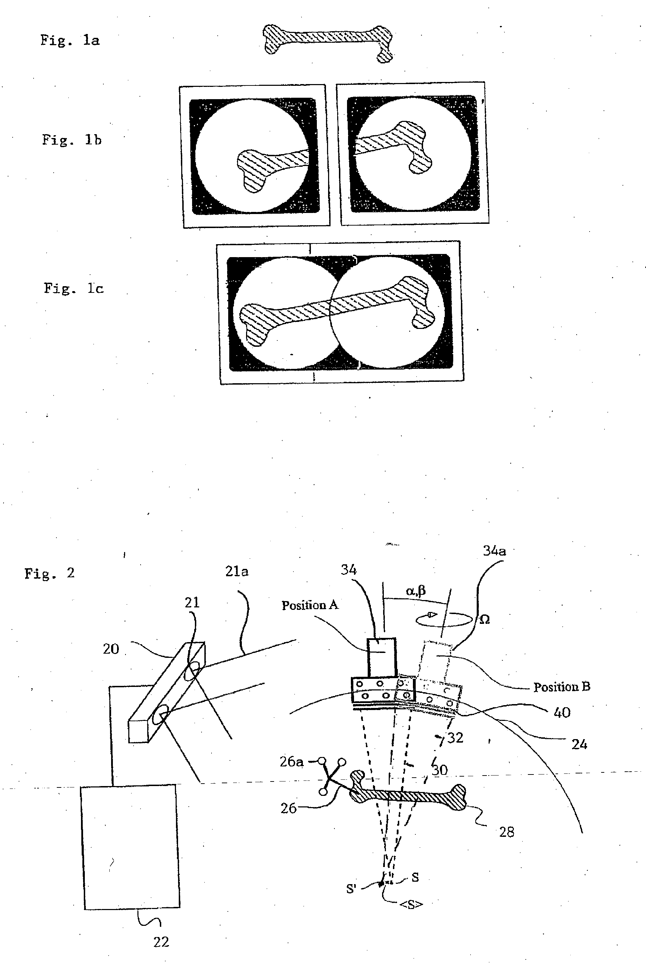

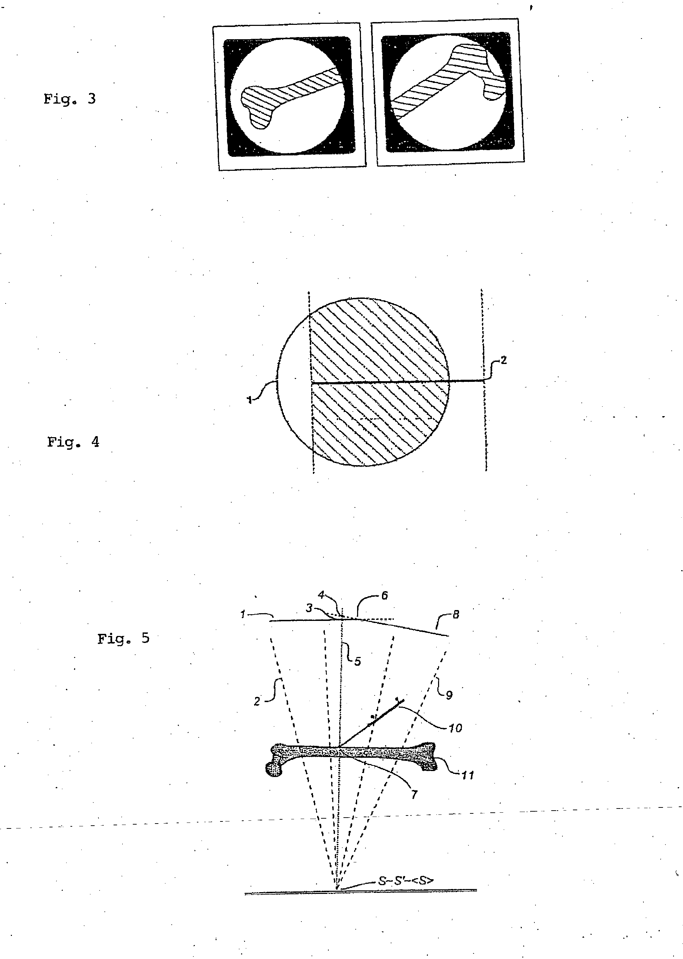

In der Fig. 3 ist eine anatomische Struktur wiedergegeben, hier ein Oberschenkelknochen, der mit einem C-Bogen mit zwei Aufnahmen an verschiedenen Positionen entlang des Körpers eines Patienten aufgenommen worden ist. Diese zwei Aufnahmen oder Röntgenbilder der anatomischen Struktur werden auf zwei Monitoren des C-Bogens dargestellt. Wie zu erkennen ist, haben die auf den verschiedenen Monitoren dargestellten Abschnitte der anatomischen Struktur gemäß Fig. 1a, wie aus Fig. 3 ersichtlich, unterschiedliche Ausrichtungen und Abbildungsvergrößerungen. Eine Orientierung anhand dieser Bildschirminhalte gemäß Fig. 3 ist reproduzierbar und realitätsentsprechend nicht möglich.

Eine zusätzliche Zielsetzung gegenüber dem Stand der Technik kann darin gesehen werden, sich dreidimensional in dem Körper oder an dem Körper eines Patienten zurechtzufinden, um operativ eine anatomische Struktur zu erreichen. Hierzu ist es nötig, dass zwei oder mehr möglichst genau überlappende Röntgenaufnahmen für eine Navigation erstellt werden, deren Überlappungsgeometrie in definierbarer Weise bekannt sein sollte. Die Zentralstrahlen dieser Röntgenaufnahmen sollten einen möglichst exakten Schnittpunkt aufweisen Die Anordnung der mehreren überlappenden Röntgenaufnahmen führt zu einer Lageinformation in drei Dimensionen, wobei die nötigen Informationen für eine Darstellung im Raum bei einer Minimierung der Abbildungsfehler aus zwei Röntgenaufnahmen zu entnehmen sind, die möglichst senkrecht zueinander aufgenommen worden sind. Dadurch, dass die Röntgenaufnahmen einander überlappen müssen, wird der effektive Abbildungsbereich, in dem mit voller dreidimensionaler Information navigiert werden kann, verkleinert. Diesbezüglich sei auf Fig. 4 verwiesen, in der ein Durchleuchtungsbild 1 dargestellt ist, das in der Ebene des Papierblattes der Fig. 4 liegt. Ein weiterer Durchleuchtungsbildbereich 2 liegt senkrecht zum Durchleuchtungsbild 1, wobei keine genaue Überlappung erkennbar ist. Der sich aus diesen beiden Bildern 1, 2 für eine Navigation ergebende verwendbare Bereich ist schraffiert dargestellt. Wie zu erkennen ist, ist der verwertbaren Bilddurchmesser aufgrund von zwei Durchleuchtungsbildern im besten Fall so groß wie der Durchmesser eines Durchleuchtungsbildes und in der Regel noch kleiner.

Insbesondere soll nämlich weiterhin eine orientiert zusammengesetzte Aufnahme von einem überlappenden Bereich verwendet werden können, um den effektiven dreidimensionalen Bereich zu vergrößern der für eine Navigation benutzt werden kann.

EP 1 782 734 bezieht sich auf ein Verfahren zur Verbesserung der Volumenrekonstruktion bei navigationsgeführten Eingriffen, denen 2D-Röntgenprojektionsaufnahmen zugrunde gelegt werden. Es wird ein Phantom mit Markern, die sowohl auf einem Röntgenbild erkennbar als auch vom Navigationssystem erfassbar ist, verwendet. Die Aufgabe einer Erfindung ist es, die Ermittlung einer Abbildungsvorschrift von mit einem Röntgengerät aufgezeichneten 2D-Projektionen des Phantoms zu einem 3D-Volumendatensatz, in dem ein Instrument geführt wird aufzuzeigen. Wesentlich ist es, dass das mit Röntgenpositiven und vom Navigationssystem erfassbaren Markern ausgestattete Phantom mit dem Untersuchungsobjekt, d.h. mit dem Patientenkörper verbunden bleibt.

US 2003/0179856 offenbart eine Vorrichtung zur Bestimmung einer Koordinatentransformation durch Mischen eines Röntgenbildes eines ersten Objekts mit einem Röntgenbild eines zweiten Objekts. Es wird eine Markerhalterung verwendet, um röntgenpositive Marker und vom Navigationssystem erfassbare Marker am Patientenkörper zu befestigen. Die Koordinaten der röntgenpositiven Marker werden in einem Koordinatensystem, das dem Navigationssystem zugewiesen ist, erfasst. Ziel der Erfindung ist es, eine Transformation zwischen dem dem Navigationssystem zugewiesenen Koordinatensystem und einem dem Röntgenbild zugewiesenen Koordinatensystem zu ermitteln.

DE 10 210 645 offenbart ein Verfahren zur Erfassung und Darstellung eines in einen Untersuchungsbereich eines Patienten eingeführten medizinischen Katheters. Das betreffende Instrument soll in einer dreidimensionalen Darstellung des Untersuchungsbereichs positionsrichtig im Volumenbild dargestellt werden. Zu diesem Zweck wird eine geeignete Transformationsmatrix genutzt, um die Koordinaten der Katheterspitze aus dem Koordinatensystem eines Navigationssystems in das eines 3D-Rekonstruktionsbilds zu transformieren. Hierbei werden im 3D-Rekonstruktionsbild und im Koordinatensystem des Navigationssystems definierte Markierungen verwendet, wobei derartige Markierungen durch Bewegen des Katheters an die Markierungspositionen definiert werden.

Die gemäß der Erfindung erzielbaren Vorteile beruhen auf dem Verfahren gemäß dem Patentanspruch 1. Zweckmäßige Verfahrensvarianten des erfindungsgemäßen Verfahrens sind den Unteransprüchen zu entnehmen.

Das erfindungsgemäße Verfahren ergibt sich bevorzugt durch die Verbringung des Röntgendetektors auf einer Kugelschale um die Röntgenquelle herum. Kann diese Verfahrensvariante ideal oder nahezu ideal verwirklicht werden, können zwei oder mehrere Röntgenaufnahmen eines anatomischen Objekts, etwa eines Knochens, mit hoher Genauigkeit zueinander orientiert und entsprechend wiedergegeben werden, da die Geometrie des Abbildungskegels zwischen der Röntgenquelle und dem Röntgendetektor in Verbindung mit dem Stereo-Aufnahmesystem so genau definiert ist, dass durch eine eindeutigen Zuordnungsvorschriften die Bildpunkte der Röntgenaufnahmen exakt oder zumindest nahezu exakt zueinander orientierbar, im Maßstab anpassbar, usw. sind.

Begleitet wird diese Vorgehensweise von dem Einsatz eines Navigationssystems mit einem Stereo-Aufnahmesystem, in der Regel einer Stereo-Kamera. Dabei sind z.B. Marker am Röntgendetektor und am abzubildenden Bereich in vorgegebener Weise angeordnet und können vom Navigationssystem etwa unter Verwendung einer Kalibrierung Raumkoordinate zugeordnet werden. Wird eine Aufnahme gemacht, wird gleichzeitig mit dieser Röntgenaufnahme das Stereoaufnahmesystem in Aktion gesetzt, um Daten über die räumliche Beziehung der Röntgenaufnahme zum abzubildenden Bereich zu erhalten. Das Navigationssystem erfasst und speichert die aktuellen Positionen der Röntgeneinheit, die die Röntgenquelle und den Röntgendetektor umfasst, und der am abzubildenden Bereich vorgesehenen Marker, die vom Navigationssystem erkannt werden. Durch eine anschließende Datenverarbeitung wird aus der Position des Detektors im Raum und aus der verzerrten Anordnung von einigen erkannten Markern, deren reale Position zueinander bekannt ist, die Position der Röntgenquelle und die Verzerrung der Abbildung des abzubildenden Bereichs ermittelt. Anschließend wird jeder Position im Koordinatensystem des abzubildenden Bereichs bzw. des anatomischen Objekts ein projizierter Punkt des angezeigten Röntgenbildes zugewiesen. Die Projektionslinien verlaufen dabei von der relativen Position aus, welche die Röntgenquelle zum Zeitpunkt der Aufnahme im Verhältnis zum abzubildenden Bereich hat.

Um erforderliche Justierungsmaßnahmen zu ermöglichen, so dass sich keine oder möglichst geringe Ortsänderung der Röntgenquelle zwischen aufeinander folgenden Aufnahmen ergeben, wird gemäß einer bevorzugten Ausführungsform das Stereo-Aufnahmesystem verwendet, um anzuzeigen, welchen Positionierungsmaßnahmen der Röntgendetektor zu unterziehen ist, damit eine zweite Quellenposition der Röntgenquelle zur Erzeugung der zweiten Aufnahme wenig von der ersten Quellenposition abweicht oder mit dieser übereinstimmt.

Dabei ist anzumerken, dass die Aufnahmen nicht einfach zum Beispiel durch geometrische Verzerrungen des Bildmaterials aneinandergehängt werden können, da dann die exakte 3-dimensionale eins-zu-eins Zuordnung zwischen Strahlen im Gegenstandsraum und Abbildungspunkten aufgehoben werden würde und jedem Gegenstandspunkt immer mindestens zwei Bildpunkte zugeordnet wären, was zum Beispiel bedeuten würde, dass ein eingebrachter dritter navigierter Gegenstand, wie z.B. ein Pointer, im Überlappungsbereich der Bilder doppelt oder gestreckt bzw. gestaucht dargestellt werden würde.

Eine automatisierte Positionierungskorrektur für die Röntgenquelle kann auch erzielt werden, wenn die Aufnahmeanordnung zur Ausführung des Verfahrens gemäß der Erfindung eine motorische oder eine mechanisch-einschränkende Anordnung umfasst, die die Positionierungsmaßnahmen automatisch umsetzt oder eine Optimierung der Lage der Röntgenquelle relativ zu der vorherigen Lage der Röntgenquelle bei der zuvor durchgeführten Röntgenaufnahme vornimmt, wobei die nachfolgende bzw. zweite Röntgenaufnahme mit einer möglichst geringen Verstellung de Röntgenquellenposition durchgeführt werden könnte.

Generell wird es durch die koordinierte Darstellung der verschiedenen Bildpunkte der Röntgenaufnahmen relativ zu einem gemeinsamen Referenzpunkt unter Berücksichtigung der Verzerrungen der Röntgenaufnahmen relativ zu dem Referenzpunkt möglich, die Röntgenaufnahmen bzw. die abzubildenden Bereiche zusammenhängend, zueinander orientiert und maßstabsangepasst wiederzugeben insbesondre um diese Aufnahmen für die Navigation zu verwenden.

Zwei oder mehr solcher zusammengesetzten Aufnahmen eines überlappenden Bereiches, die möglichst zueinander senkrecht stehen oder zumindest einen von null verschieden Winkel einnehmen können insbesondere verwendet werden um auf einem durchgehenden Bereich mit voller dreidimensionaler Information zu navigieren, der deutlich größer ist als der Bildbereich eines Röntgengerätes ist.

Gemäß dem erfindungsgemäßen Verfahren kann eine Röntgenabbildung eines aufzunehmenden Objektes vom Navigationssystem zugeordneten Markerpositionen registriert und als Referenzbild für eines oder mehrere nachfolgende Röntgenaufnahmen herangezogen werden. Daraufhin wird der Röntgendetektor aus einer ersten Position verbracht, wobei die Bewegung des Röntgendetektors entlang der Oberfläche einer Kugelschale erfolgt, wobei die Röntgenquelle im Zentrum der Kugelschale liegt. Die relativen Positionen des Röntgendetektors und der Röntgenquelle sind dabei durch die bei der ersten Aufnahme erhaltenen Rauminformationen bekannt, die anhand der zuvor aufgezeigten Kalibrierung auch entzerrt worden sind. Nun ist es wesentlich, dass der Röntgendetektor so in eine zweite Position verbracht wird, dass die neue Position der Röntgenquelle mit der alten zusammenfällt, oder dass eine Abweichung zwischen der Position der Röntgenquelle bei der ersten Aufnahme und der Position der Röntgenquelle bei der zweiten Aufnahme eine bestimmte Abweichung nicht überschreitet. Vorteilhaft ist eine Abweichung in einer Größenordung von wenigen Zentimetern, insbesondere von 1 cm oder darunter tolerierbar. Ist es möglich, die Positionen der Röntgenquelle bei der ersten und bei der zweiten Aufnahme übereinstimmen zu lassen, so lässt sich eine ideale Abbildungsvorschrift verwirklichen. Ist eine tolerierbare Positionsdifferenz zwischen der Position der Röntgenquelle bei der ersten Aufnahme und der Position der Röntgenquelle bei der zweiten Aufnahme eingerichtet worden, wird eine geeignete dritte bzw. weitere Quellenposition aus den beiden Quellenpositionen errechnet, die insbesondere die gemittelte Quellenposition ist oder zum Beispiel auch eine, mit den einzelnen Abbildungsfehlern gewichtete, mittlere Quellenposition seien kann. Ausgehend von dieser errechneten dritten bzw. weiteren Quellenposition kann ebenfalls eine Abbildungsvorschrift für beide Projektionskegel und damit für beide Röntgenaufnahmen errechnet werden. Die Bildinformationen der Röntgenbilder können dann aneinander angepasst im Maßstab, in der Orientierung und sämtlichen anderen für eine getreue Abbildung erforderlichen Parametern mit einem größtmöglichen Informationsgehalt wiedergegeben werden.

Vorteilhaft überlappen die Aufnahmen. Sollten die Aufnahmen nicht überlappen, wird ein nicht erfasster Bereich zwischen den Aufnahmen nicht oder beispielsweise schwarz dargestellt. Der nicht erfasste Bereich kann auch durch die Durchführung einer oder mehrer weiterer Aufnahmen aufgefüllt werden. Auch direkt aneinander anschließende Aufnahmen, die keine Überlappung aufwesen, lassen sich gemäß der Erfindung bearbeiten, da auch dies durch die Bereitstellung einer Quellenposition ermöglicht wird, wobei die Aufnahmen einer Quellenposition ermöglichst wird, wobei die Aufnahmen in einer Kugelschale um die den Aufnahmen gemeinsame Quellenposition verdreht werden können.

Um weitere Bild- bzw. Drientierungsinformationen für einen Operateur bzw. Arzt bereit zu stellen, ist es bevorzugt, nach der Fertigung einer ersten und einer zweiten Aufnahme gemäß der Erfindung das Röntgengerät in eine andere Stellung zu verfahren, um dort in einem anderen Winkel zu dem aufzunehmenden Bereich wiederum wenigstens eine Aufnahme oder zwei Aufnahmen nach dem erfindungsgemäßen Verfahren aufzunehmen. Dabei ist es besonders vorteilhaft, wenn der zweite Satz einer ersten und einer zweiten Aufnahme in einem bestimmten Winkel, bevorzugt um die 90°, zum ersten Satz einer ersten und zweiten Aufnahme erzielt worden ist, da derart optimierte räumliche Bildinformationen erhalten werden können. Es ist jedoch auch möglich, ab geringen Winkeln erfindungsgemäß vorzugehen, etwa ab einem minimalen Winkel von wenigstens ca. 30 °, bevorzugt aber ab einem Winkel von ca. 60 °. Dabei ist es besonders vorteilhaft, wenn der zweite Satz einer ersten und einer zweiten Aufnahmen in einem bestimmten Winkel, bevorzugt um die 90 °, typischerweise größer als 60 ° oder auch größer als 30 ° zum ersten Satz einer ersten und zweiten Aufnahmen erzielt worden ist. Die jeweils überlappenden Aufnahmen der beiden voneinander eventuell distanzierten und/oder zueinander verwinkelt aufgenommenen Sätze von Aufnahmen können dann zueinander gemäß der Erfindung registriert und orientiert werden, um zu einem oder mehreren zusammenhängenden Bildern auf einem oder mehreren Bildschirmbereichen oder Bildschirmen zusammengesetzt zu werden. Dabei werden mittels der Stereokamera und den von dieser erfassten Markern räumlichen Zuordnungen zwischen den Bildinformationen der Röntgenaufnahmen rechnerisch erstellt.

Natürlich gehören zur Erfindung auch Programme, die das erfindungsgemäße Verfahren realisieren sowie Datenträger, die entsprechende Programme zur Ausführung auf einen Computer zur Verwirklichung des erfindungsgemäßen Verfahrens in digitaler Form speichern.

Nachfolgend wird die vorliegende Erfindung anhand einer bevorzugten Ausführungsform unter Bezugnahme auf die beigefügten Darstellungen näher beschrieben, wobei weitere Merkmale, Vorzüge sowie Zielsetzungen gemäß der Erfindung offenbart werden. In den Darstellungen zeigen:
- Fig. 1a bis 1c: eine anatomische Struktur (Fig. 1a), die nicht überlappend aufgenommen wurde und einerseits orientiert und korrekt skaliert dargestellt wird (Fig. 1b) und andererseits in einer überlappenden Darstellung nach der Erfindung (Fig. 1c) aufgenommen und wiedergegeben wird;
- Fig. 2: eine Aufnahmeanordnung zur durchführung des Verfahrens gemäß der Erfindung, bzw. einer Variante davon;
- Fig. 3: eine anatomische Struktur nach Fig. 1a in zwei nicht orientierten Darstellungen auf einem Bildschirm (Navigation, EDV) gemäß dem Stand der Technik;
- Fig. 4: zwei überlappend dargestellte Aufnahmen für die Navigation in schematischer Weise gemäß dem Stand der Technik, wobei die Aufnahmeebenen im wesentlichen aufeinander senkrecht stehen, und
- Fig. 5: einen teilweise dargestellten Verfahrenablauf zur Registrierung von zwei Aufnahmen eines abzubildenden Beeichs.

Die Fig. 1a zeigt eine anatomische Struktur in der Form eines menschlichen Oberschenkelknochens. Es gilt nun, diese anatomische Struktur mittels eines Röntgengerätes, insbesondere eines sog. C-Bogens, aufzunehmen und die dabei erzeugten Durchleuchtungsbilder so wiederzugeben, dass es dem Betrachter ermöglicht wird, aus den bereitgestellten Bildinformationen den größtmöglichen Nutzen zu ziehen. D.h., die dargestellten Bildinhalte müssen genau sein, zueinander passen sowie nach Möglichkeit in Bezug auf ihre Abmessungen, Dimensionen, Verwinklungen, usw. aneinander angepasst sein oder wenigstens erkennbar gemacht sein.

Werden die Röntgenaufnahmen beispielsweise in zwei Anzeigebereichen wiedergegeben, kann, wie in Fig. 1b gezeigt, eine Registrierung der beiden Bildinhalte der beiden Aufnahmen, die das Röntgengerät gemacht hat, vorgenommen werden, anhand derer sich der Betrachter orientieren kann. Ein Vergleich mit der Fig. 3 macht den Unterschied zum Stand der Technik sofort deutlich. Während gemäß der Erfindung eine registrierte Darstellung der Bildinhalte erfolgt, wobei die Bildinhalte bezüglich ihrer Abmessungen, Vergrößerungen, etc. zueinander passen, kann gemäß dem Stand der Technik von einer genauen Orientierung anhand der Abbildungen keine Rede sein (siehe Fig. 1a, 3).

Eine der Fig. 1b entsprechende Darstellung ist gemäß Fig. 1c vor Augen geführt. Hier werden die beiden Aufnahmen, wie sie auch gemäß Fig. 1b in zwei Anzeigebereichen abgebildet werden, gemäß der Erfindung in unmittelbar aneinander anschließender Weise dargestellt, so dass erkennbar ist, dass sich die Aufnahmen, die gemäß der Erfindung aufgenommen bzw. wiedergegeben werden, überlappen, wobei ein Bildbereich der einen Aufnahme den Überlappungsbereich ergeben kann, der mit einem Bildbereich der anderen Aufnahme zur Überlappung gebracht wird, so dass die dargestellte anatomische Teilstruktur im linken Bildschirminhalt an die Teilstruktur im rechten Bildschirminhalt nahtlos anschließt.

In den Fig. 2 und 5 wird der prinzipielle Aufbau eines navigierten Röntgenaumahmesystems dargestellt, wie er zur Ausführung des Verfahrens nach der vorliegenden Erfindung zum Einsatz gelangen könnte. In der Fig. 2 ist ein Navigationssystem prinzipiell dargestellt, das unter anderem eine Stereo-Kamera 20 mit einem Paar von Objektiven 21 umfasst. Die Stereo-Kamera ist an eine Datenverarbeitungsanlage 22 angeschlossen. Die Objektive 21 der Stereo-Kamera, beispielsweise einer Infrarot-Stereo-Kamera, weisen jeweilige Erfassungswinkel 21a bzw. entsprechende Erfassungsbereiche auf, innerhalb derer die Existenz von Markern 26a erfasst und positionsmäßig verarbeitet werden kann.

An einem Röntgendetektor 34, oder an einem anderen Gegenstand bekannter Geometrie, an dem sich Röntgenmarker befinden und der sich im Strahlengang befindet (nicht gezeigt), sind Marker 26a vorgesehen, der Röntgendetektor 34 liegt einer Röntgenquelle S, gegenüber. Zwischen dem Röntgendetektor 34 und der Röntgenquelle S erstreckt sich ein Aufnahmekegel 30, in dem ein abzubildendes Objekt 28, hier ein Oberschenkelknochen 28, durch eine bzw. mehrer Röntgenaufnahmen erfasst und abgebildet werden soll. An dem Oberschenkelknochen 28 sind ebenfalls mehreren Marker 26a in der Form eines Markerkits 26 mit mehreren Markern 26a positionsfest angeordnet.

An dem Röntgendetektor 34 kann zum Zweck der Bildentzerrung und Registrierung eine Kalibrierungsanordnung 40 angeordnet sein.

Mechanisch bzw. elektromagnetisch können Positionsänderungen sowohl der Quelle als des Röntgendetektors ebenfalls ermittelt werden. Beispielsweise können induktiv arbeitende Sensoren eingesetzt werden, oder Schrittgeber könne in beliebigen Raumrichtungen Schrittpulse abgeben, die ebenfalls gemäß der Erfindung für eine entsprechende Bildverarbeitung herangezogen werden können.

Da die prinzipielle Funktionsweise eines derartigen Röntgengerätes in Verbindung mit einem Navigationssystem, das voranstehend bereits erläutert wurde, wird hierauf lediglich Bezug genommen.

Bei der Darstellung gemäß Fig. 2 wird nun darauf geachtet, dass der Röntgendetektor 34 entlang einer Kugelschale 24 gegenüber dem Oberschenkelknochen 28 in eine andere Position verbracht wird, die durch das Bezugszeichen 34a angedeutet wird. Während in der ersten Position A der Röntgendetektor 34 den Abbildungskegel 30 aufweist, der durch die gegenüberliegende Röntgenquelle S vorgegeben wird, hat der Röntgendetektor 34 in der zweiten Position B (angedeutet als 34a) zur Aufnahme einer zweiten Röntgenaufnahme einen Projektionskegel 32, an dessen spitzem Ende die geringfügig ortsversetzte Röntgenquelle S' liegt. Natürlich stimmt die Röntgenquelle S mit der Röntgenquelle S' überein, ist aber durch die Verbringung des Röntgendetektors 34 auf die Position B leicht ortsversetzt. Idealerweise würde sich die Röntgenquelle S an derselben Stelle befinden, nachdem der Röntgendetektor in die Position B verbracht worden ist, was in der Praxis aber schwer möglich sein wird. Die Winkel α und β beschreiben die Winkelveränderung zwischen der Verbindungsgerade die durch die Röntgenquelle S und dem Röntgendetektor in der Position A definiert wird sowie der Verbindungsgerade die durch die Röntgenquellenposition S' und dem Röntgendetektor 34 in seiner Position B definiert wird. Beispielsweise entspricht α einer Winkelveränderung in der ebene des Blattes und β einer Winkelveränderung senkrecht dazu.. Der Winkel Ω entspricht einer Rotation des Röntgendetektors um die Achse, die durch die Verbindungsgerade zwischen dem Röntgendetektor 34, 34a und der Röntgenquelle S, S' entsteht.

Bei der Verbringung des Röntgendetektors aus der Position A in die Position B verbleibt die Position der Röntgenquelle S nahe bei dieser Position und wird lediglich in eine leicht abweichende Position S' verbracht. Hierdurch kann eine mittlere Position der Röntgenquelle <S> ermittelt werden, oder auch eine andere geeignete Position nahe der gemittelten Position. Mit dieser Position <S> kann eine eindeutige Abbildungsvorschrift erstellt werden, mit der sämtliche Bildpunkte, die in der Position A aufgenommen worden sind sowie sämtliche Bildpunkte die in der Position B aufgenommen worden sind, gemeinsam zueinander orientiert, koordiniert sowie maßstabsgetreu abgebildet werden können. Der dabei entstehende Ungenauigkeit für die Navigation kann dabei bestimmt werden (z.B. durch rein geometrische Überlegungen bzw. Berechnungen, die auch von der EDV der Navigation durchgeführt werden können) oder durch einen Vergleich von im Bild detektierten Markern bekannter Position, mit der aus der Registrierung stammenden theoretischen Position, und kann dann Beispielsweise dem Benutzer angezeigt werden bzw. dazu benutzt werden um die zweite Aufnahmen im Sinne dieses Verfahren zuzulassen oder zu verwerfen.

Die in der vorliegenden Offenbarung angegebenen Verfahrensvarianten haben insbesondere gemeinsam, dass durch die Verwendung von miteinander in Beziehung setzbaren Abbildungskegeln zwischen den Röntgenquellen und den Röntgendetektoren, die ermittelten Bilddaten der zwei oder mehreren Abbildungen mit einem minimalen Fehler durch eine gemeinsame Abbildungsvorschrift verarbeitet werden können, um diese auf einem, zwei oder beliebig vielen Bildschirmausschnitten oder Bildschirmen zueinander orientiert, passend im Maßstab sowie mit weiteren Orientierungshilfen für eine Bedienungsperson, etwa einen Chirurgen, zur Verfügung gestellt werden.

Unter Verwendung der im Stand der Technik unter der Bezeichnung "MEPUC" bekannten Software der Anmelderin kann erreicht werden, dass Aufnahmen eines abzubildenden Bereichs innerhalb tolerabler, zu bestimmender Grenzen aufgenommen werden. Diese Software und Erläuterungen sind dem Fachmann zwar bekannt, sollen jedoch hier nochmals definitiv durch Bezugnahem in die vorliegende Offenbarung aufgenommen sein.

Unter Bezugnahme auf Fig. 5 wird im Überlappungsbereich von Röntgenstrahlkegeln 2, 9, ein abzubildender Bereich 11 auf die Bildebenen 1 von S aus und 8 von S' aus projiziert, wobei eine navigierte Wiedergabe in der durch die Bildebenen bzw. Bilder 1, 8 aufgespannten Abbildungsebene erzeugt wird. Dabei kann beispielsweise ein differenzierbarer Bildpunkt 7 eines Objekts, hier die Spitze eines Zeigeinstruments (Pointer), entlang eines Strahles 5 von <S> aus auf Punkte 3 und 4 in den entsprechenden Abbildungsebenen projiziert werden. Eine orientierte Wiedergabe kann aus dem Bild 1 bis zum Schnittpunkt 6 und ab dort aus Bild 8 zusammengesetzt werden, oder der jeweils gestrichelte Teil der Bilder 1, 8 wird entlang der Zentralstrahlen auf die ungestrichelten Teile projiziert. Dort werden die Bildinformationen mit jeweils 50%iger Transparenz oder Dämpfung aufaddiert, um dem Umstand Rechnung zu tragen, dass die Projektionen geringfügig (z.B. unter 1 mm) gegeneinander verschoben sind. Das gemeinsame Koordinatensystem wird mit der Annahme aufgespannt, dass <S> das gemeinsame Projektions- oder Symmetriezentrum ist.

Objekte, die mit einer nicht perfekten Überlappung von S und S' aufgenommen werden, können einen leicht diffusen Rand und/oder unscharfe Zonen enthalten, da sie auf leicht unterschiedliche Stellen des Panoramabildes für die orientierte Wiedergabe projiziert werden. Dieser diffuse Rand ist eine gute optische Anzeige für die verringerte Genauigkeit.

Wird jeweils ein definierter Bildteil benutzt, besteht ferner die Möglichkeit die Bildinformationen und die Position eines navigierten Werkzeugs im Panoramabild des abzubildenden Bereiches sowohl von S als auch von S' aus zu berechnen und das die Bildinformation sowie das Werkzeug lediglich im Überlappungsbereich in Bezug auf <S> gemittelt, entsprechend mit einer leicht vergrößerten unscharfen oder verzerrten Kontur darzustellen.

Die beiden soeben beschriebenen Wiedergabeverfahren funktionieren insbesondere auch, wenn sich die Röntgenstrahlkegel nicht überlappen. Der Bereich zwischen den Bildern kann dann z.B. schwarz dargestellt werden, wie sich dies (im Prinzip aus Fig. 1b ergibt). Die Position von navigierten Werkzeugen kann in diesem Fall immer aus den genauen Positionen der Projektionszentren berechnet werden. Der Abstand, der Maßstab und die Orientierung der Wiedergaben zueinander ergeben sich aufgrund der Position von <S>, dem gemeinsamen Zentrum.

Gemäß der Erfindung können auch mehr als zwei Bilder zusammengefasst werden, insbesondere wenn sich der Röntgendetektor auf einer Kugelschale um S, der Röntgenquelle, befindet, wenn eine Aufnahme gemacht wird. Diese Kugelschale wird dann mit einer Projektion auf die 2D-Ebene des Monitors umgerechnet bzw. transformiert. Derartige Wiedergaben können auch in anderen Koordinatensystemen erzeugt werden. Wesentlich ist, dass die Röntgenquelle oder im Symmetriezentrum des Koordinatensystems liegt und eine eindeutige Abbildungsfunktion existiert, mit der die Bildpunkte der zusammenzufügenden Röntgenaufnahmen aneinander angepasst werden können.

Mit den unter Bezugnahme auf die Figuren 2 und 5 beschriebenen Verfahren lässt sich ein Satz von Aufnahmen mit einer ersten und einer zweiten Röntgenaufnahme erstellen. Weiterhin lässt sich ein zweiter Satz mit einer ersten und einer zweiten Röntgenaufnahme gemäß der Erfindung erstellen. Die Position der Verbindungsgraden von <S> zum Mittelpunkt aller für den ersten Satz benutzten Detektorpositionen kann dann zu der entsprechenden Verbindungsgraden zweiten Satzes verwinkelt, insbesondere um 90° und/oder distanziert aufgenommen werden. Über von der Stereokamera bei der Herstellung der jeweiligen Aufnahmesätze gespeicherten Markerpositionen können dann die Bildinformationen der Aufnahmesätze zueinander in eine lineare Relation gesetzt werden. Natürlich lassen sich noch weitere Sätze von zwei oder mehr Röntgenaufnahmen entsprechend herstellen und zueinander orientiert bzw. registriert werden, um diese überlagert auf einem oder mehreren Bildschirmen wiederzugeben.

Insgesamt lässt sich gemäß der Erfindung und deren Ausführungsformen der insbesondere für eine dreidimensionale Navigation verwendbare Bereich ganz erheblich vergrößern, d.h., insbesondere der Bereich, der aus Fig. 4 hervorgeht.

## Patentansprüche

1. Verfahren zur orientierten Darstellung von, insbesondere überlappenden, Aufnahmen eines in wenigstens zwei Aufnahmeschritten aufgenommenen, abzubildenden Bereichs (11), insbesondere einer anatomischen Struktur wie ein Oberschenkelknochen (28), mit einem Navigationssystem, wobei das Navigationssystem folgende Merkmale umfasst:
- einen Röntgendetektor (34, 34a) mit einer diesem gegenüber angeordneten Röntgenquelle (S, S'), die zueinander in einem vorgebbaren Verhältnis gemeinsamen und zu dem abzubildenden Bereich bewegbar sind;
- ein Stereo-Aufnahmesystem (20, 21, 22), etwa einer Stereokamera (20);
- mehrere Markereinrichtungen, die dem Röntgendetektor (34, 34a) und dem abzubildenden Bereich zugeordnet sind, und die wenigstens teilweise durch das Stereo-Aufnahmesystem erfassbar sind;
wobei das Verfahren durch die nachfolgenden Schritte gekennzeichnet ist:
a) eine erste Aufnahme des abzubildenden Bereichs (11) wird durch das Röntgengerät in einer ersten Position (S, A) ausgelöst, wobei ein erstes Steuersignal abgegeben wird;
b) durch das erste Steuersignal wird das Stereo-Aufnahmesystem veranlasst, erste Markerpositionen der mehreren Markereinrichtungen am Röntgendetektor (34, 34a) und am abzubildenden Bereich (11) zu erfassen;
c) Daten zu den ersten Markerpositionen werden gespeichert;
d) die Daten zu den ersten Markerpositionen werden rechnergestützt in ein Verhältnis zu Bildpunkten der Röntgenaufnahme gesetzt, um die Röntgenaufnahme zu registrieren;
e) eine erste relative Position von der Röntgenquelle (S, S') zum Röntgendetektor (34, 34a) wird ermittelt und gespeichert;
f) mit Hilfe der Registrierung wird die Position der Röntgenquelle im Koordinatensystem des abzubildenden Bereichs (11) bestimmt und gespeichert
g) der Röntgendetektor (34, 34a) wird zusammen mit der Röntgenquelle relativ zum abzubildenden Bereich in eine zweite Position (S', B) für eine zweite Aufnahme verbracht, wobei während der Verbringung Positionsänderungen des Röntgendetektors (34, 34a) erfasst und mittels der ersten relativen Position eine zweite relative Position der Röntgenquelle (S, S') bestimmt wird;
h) eine zweite Aufnahme wird an der zweiten Position (S', B) ausgelöst, wobei ein zweites Steuersignal abgegeben wird;
i) durch das zweite Steuersignal wird das Stereo-Aufnahmesystem (20, 21, 22) veranlasst, zweite Markerpositionen der Markereinrichtungen am Röntgendetektor (34, 34a) und am abzubildenden Bereich (11) zu erfassen;
j) die Daten zu den zweiten Markerpositionen werden rechnergestützt in ein Verhältnis zu Bildpunkten der Röntgenaufnahme gesetzt, um die zweite Röntgenaufnahme zu registrieren;
k) aus der zweiten relativen Position und aus der ersten relativen Position wird eine weitere relative Position (<S>) der Röntgenquelle ermittelt;
l) mittels der weiteren relativen Position (<S>) werden die Aufnahmen registriert und überlagert dargestellt.

2. Verfahren nach Anspruch 1, **dadurch gekennzeichnet, dass** die Positionsänderungen des Röntgendetektors (34, 34a) mittels des Navigationssystems und/oder mechanisch festgestellt erden.

3. Verfahren nach Anspruch 1 oder 2, **dadurch gekennzeichnet, dass** eine Differenz zwischen der ersten und der zweiten Position der Röntgenquelle bestimmt wird.

4. Verfahren nach Anspruch 3, **dadurch gekennzeichnet, dass** bestimmt wird, ob die Differenz einen Differenzmaximalwert überschreitet.

5. Verfahren nach den Ansprüchen 1 bis 4, **dadurch gekennzeichnet, dass** die gemeinsame Position durch räumliche Mittelung oder eine gewichtete Berechnung bzw. eine Mittelung errechnet wird.

6. Verfahren nach den Ansprüchen 1 bis 5, **dadurch gekennzeichnet, dass** die zweite Aufnahme bzw. eine weitere Aufnahme unterdrückt oder wiederholt wird, wenn die Differenz den Differenzmaximalwert überschreitet.

7. Verfahren nach einem der voranstehenden Ansprüche, **dadurch gekennzeichnet, dass** die Verbringung des Röntgendetektors (34, 34a) so durchgeführt wird, dass die zweite Position (B) gleich oder ähnlich zu der ersten Position (A) des Röntgendetektors (34, 34a) bei einer Röntgenaufnahme ist.

8. Verfahren nach einem der voranstehenden Ansprüche, wobei das Stereo-Aufnahmesystem (20, 21, 22) basierend auf einer ersten Quellenposition der Röntgenquelle, die berechnet wird, bei der ersten Aufnahme ermittelt, welchen Positionierungsmaßnahmen der Röntgendetektor (34, 34a) zu unterziehen ist, damit eine zweite Quellenposition der Röntgenquelle (S, S') zur Erzeugung der zweiten Röntgenaufnahme wenig von der ersten Position der Röntgenquelle abweicht oder mit dieser übereinstimmt.

9. Verfahren nach einem der voranstehenden Ansprüche, wobei erforderliche Positionierungsmaßnahmen mittels einer Anzeigeeinrichtung einer Bedienungsperson angezeigt werden.

10. Verfahren nach einem der voranstehenden Ansprüche, wobei die Aufnahmeanordnung eine motorische Anordnung umfasst, die die Positionierungsmaßnahmen manuell gesteuert oder automatisch umsetzt.

11. Verfahren nach einem der voranstehenden Ansprüche, wobei weitere Röntgenaufnahmen von anderen Verbringungspositionen her gemacht werden und entsprechend der zweiten Röntgenaufnahme an die erste Röntgenaufnahme oder an eine aufgrund dieser erzeugte Gesamtdarstellung in angepasster Form angegliedert werden.

12. Verfahren nach einem der voranstehenden Ansprüche, wobei ein erster Satz von Aufnahmen, der die erste und die zweite Aufnahme umfasst, relativ zu einer ersten gemeinsamen Position erstellt wird, und ein zweiter Satz von Aufnahmen, der eine weitere erste und zweite Aufnahme enthält, in einer zweiten gemeinsamen Position, die von der ersten verschieden ist, erstellt wird, wobei die gemeinsamen Positionen um einen Winkel zueinander verdreht sind und/oder voneinander distanziert sind.

13. Verfahren nach Anspruch 12, wobei der Winkel einen Minimalwert überschreitet, der wenigstens etwa 30°, bevorzugt etwa 60 ° und insbesondere wenigstens in etwa 90° beträgt.

14. Verfahren nach einem der voranstehenden Ansprüche, wobei die Steuersignale vom Röntgengerät oder dem Navigationssystem aufgrund einer die Auslösung einer Aufnahme ergebenden Bildanalyse erzeugt werden.

## Claims

1. A method for oriented display of in particular overlapping images of a region (11) to be imaged, in particular of an anatomical structure such as a femoral bone (28), which has been imaged in in at least two imaging steps using a navigation system, wherein the navigation system comprises the following features:
- an x-ray detector (34, 34a) having an x-ray source (S, S') disposed opposite to it, which are movable in a presettable relationship relative to each other and relative to the region to be imaged;
- a stereo-acquisition system (20, 21, 22) such as a stereo camera (20);
- a plurality of marker devices which are associated with the x-ray detector (34, 34a) and the region to be imaged and which are at least partially detectable by the stereo-acquisition system;
- wherein the method is **characterised by** the following steps:
a) a first image of the region (11) to be imaged is triggered by the x-ray device at a first position (S, A), outputting a first control signal;
b) the stereo-acquisition system (20) is made by the first control signal to acquire first marker positions of the plural marker devices (26a) on the x-ray detector (34) and the region to be imaged;
c) data about the first marker positions is stored;
d) the data about the first marker positions is related, using a computer, to image points of the x-ray image in order to register the x-ray image;
e) a first relative position of the x-ray source (S, S') relative to the x-ray detector (34, 34a) is determined and stored;
f) using the registration, the position of the x-ray source in the coordinate system of the region (11) to be imaged is determined and stored;
g) the x-ray detector (34, 34a) is moved, relative to the region to be imaged and together with the x-ray source, into a second position (S', B) for a second image, wherein positional changes of the x-ray detector (34, 34a) are acquired during the movement and using the first relative position, a second relative position of the x-ray source (S, S') is determined;
h) a second image is triggered in the second position (S', B), outputting a second control signal;
i) the stereo-acquisition system (20, 21, 22) is made, by the second control signal, to acquire second marker positions of the marker devices on the x-ray detector (34, 34a) and on the region (11) to be imaged;
j) data about the second positions is related, using a computer, to image points of the x-ray image, in order to register the second x-ray image;
k) from the second relative position and the first relative position, a second relative position (<S>) of the x-ray source is determined;
l) via the further relative position (<S>), the images are registered and displayed in an overlapping manner.

2. The method according to claim 1, **characterised in that** the positional changes of the x-ray detector (34, 34a) are determined using the navigation system and/or mechanically.

3. The method according to claim 1 or 2, **characterised in that** a difference between the first and the second position of the x-ray source is determined.

4. The method according to claim 3, **characterised in that** it is determined whether the difference exceeds a maximum difference value.

5. The method according to one of claims 1 to 4, **characterised in that** that the common position is calculated by spatial averaging or a weighted calculation and/or an averaging.

6. The method according to one of claims 1 to 5, **characterised in that** the second image and/or a further imaging is suppressed or repeated if the difference exceeds the maximum difference value.

7. The method according to one of the preceding claims, **characterised in that** the movement of the x-ray detector (34, 34a) is performed such that the second position (B) is equal or similar to the first position (A) of the x-ray detector (34, 34a) during an x-ray imaging.

8. The method according to one of the preceding claims, wherein the stereo acquisition system (20, 21, 22) determines during the first image, based on a first source position of the x-ray source what positioning measures the y-ray detector (34, 34a) has to be subjected to in order for a second source position of the x-ray source (S, S') for generating the second x-ray imaging to deviate little from the first position of the x-ray source or to match it.

9. The method according to one of the preceding claims, wherein the necessary positioning measures are displayed to an operator using a display device.

10. The method according to one of the preceding claims, wherein the imaging arrangement comprises a motorized arrangement which implements the positioning measures in a manually controlled manner or automatically.

11. The method according to one of the preceding claims, wherein further x-ray images are taken from other movement positions und are attached to the first x-ray image or a total display generated on the basis of the first x-ray image according to the second x-ray image in an adapted form.

12. The method according to one of the preceding claims, wherein a first set of images comprising the first and the second image is generated relative to a first common position and a second set of images comprising the first and second image is generated in a second common position different from the first one, wherein the common positions are rotated relative to one another and/or spaced from one another by an angle.

13. The method according to claim 12, wherein the angle exceeds a minimum value which amounts to at least about 30°, preferably about 60° and in particular at least about 90°.

14. The method according to any one of the preceding claims, wherein the control signals are generated by the x-ray device or the navigation system due to an image analysis resulting in the triggering of an image.

## Revendications

1. Procédé de représentation orientée de prises de vues, en particulier se chevauchant, d'une région (11) à représenter, notamment d'une structure anatomique comme un fémur (28), photographiée en au moins deux étapes de prise de vue avec un système de navigation, où le système de navigation comporte les caractéristiques suivantes :
- un détecteur de rayons X (34, 34a) avec une source de rayons X (S, S') disposée en regard de celui-ci, aptes à être déplacés l'un par rapport à l'autre et par rapport à la région à représenter selon un rapport pré-déterminable;
- un système de prise de vue stéréoscopique (20, 21, 22), par exemple un appareil stéréoscopique (20) ;
- plusieurs dispositifs de marquage associés au détecteur de rayons X (34, 34a) et à la région à représenter, et qui sont au moins partiellement détectables par le système de prise de vue stéréoscopique ;
où le procédé est **caractérisé par** les étapes suivantes :
a) une première prise de vue de la région à représenter (11) est déclenchée par l'appareil de radiographie dans une première position (S, A), un premier signal de commande étant alors émis ;
b) le premier signal de commande ordonne au système de prise de vue stéréoscopique de détecter des premières positions de marquage de la pluralité de dispositifs de marquage sur le détecteur de rayons X (34, 34a) et sur la région à représenter (11) ;
c) des données concernant les premières positions de marquage sont enregistrées ;
d) les données concernant les premières positions de marquage sont mises en relation à l'aide d'un ordinateur avec des points d'image de la prise de vue radiographique afin d'enregistrer la prise de vue radiographique ;
e) une première position relative de la source de rayons X (S, S') par rapport au détecteur de rayons X (34, 34a) est déterminée et sauvegardée ;
f) la position de la source de rayons X dans le système de coordonnées de la région à représenter (11) est déterminée à l'aide de l'enregistrement et sauvegardée ;
g) le détecteur de rayons X (34, 34a) et la source de rayons X (S, S') sont déplacés relativement à la région à représenter vers une deuxième position (S', B) pour une deuxième prise de vue, où, au cours du déplacement, des changements de position du détecteur de rayons X sont détectés et une deuxième position relative de la source de rayons X (S, S') est déterminée au moyen de la première position relative :
h) une deuxième prise de vue est déclenchée dans la deuxième position (S', B), un deuxième signal de commande étant alors émis ;
i) le deuxième signal de commande ordonne au système de prise de vue stéréoscopique (20, 21; 22) de détecter des deuxièmes positions de marquage des dispositifs de marquage sur le détecteur de rayons X (34, 34a) et sur la région à représenter (11) ;
j) les données concernant les deuxièmes positions de marquage sont mises en relation à l'aide d'un ordinateur avec des points d'image de la prise de vue radiographique afin d'enregistrer la deuxième prise de vue radiographique ;
k) une autre position relative (<S>) de la source de rayons X est déterminée à partir de la deuxième position relative et de la première position relative ;
l) les prises de vues sont enregistrées au moyen de l'autre position relative (<S>) et représentées de manière superposée.

2. Procédé selon la revendication 1, **caractérisé en ce que** les changements de position du détecteur de rayons X (34, 34a) sont détectés au moyen du système de navigation et/ou mécaniquement.

3. Procédé selon la revendication 1 ou 2, **caractérisé en ce qu'**est déterminée une différence entre la première et la deuxième position de la source de rayons X.

4. Procédé selon la revendication 3, **caractérisé en ce que** l'on détermine si la différence dépasse une valeur de différence maximale.

5. Procédé selon les revendications 1 à 4, **caractérisé en ce que** la position commune est déterminée par calcul de la moyenne spatiale ou par un calcul pondéré, respectivement par un calcul de moyenne.

6. Procédé selon les revendications 1 à 5, **caractérisé en ce que** la deuxième prise de vue, respectivement une autre prise de vue est empêchée ou renouvelée lorsque la différence dépasse une valeur de différence maximale.

7. Procédé selon l'une quelconque des revendications précédentes, **caractérisé en ce que** le déplacement du détecteur de rayons X (34, 34a) s'effectue de manière à ce que, lors d'une prise de vue radiographique, la deuxième position (B) est égale ou similaire à la première position (A) du détecteur de rayons X (34, 34a).

8. Procédé selon l'une quelconque des revendications précédentes, où lors de la première prise de vue, le système de prise de vue stéréoscopique (20, 21, 22) détermine à partir de la première position source de la source de rayons X calculée, quelles mesures de positionnement doivent être appliquées au détecteur de rayons X (34, 34a) pour que la deuxième position source de la source de rayons X (S, S') pour générer la deuxième prise de vue radiographique ne diffère que de peu de la première position de la source de rayons X ou coïncide avec elle.

9. Procédé selon l'une quelconque des revendications précédentes, où les mesures de positionnement nécessaires sont indiquées à un opérateur au moyen d'un dispositif d'affichage.

10. Procédé selon l'une quelconque des revendications précédentes, où le dispositif de prise de vue comporte un dispositif moteur qui met en oeuvre les mesures de positionnement sous contrôle manuel ou automatiquement.

11. Procédé selon l'une quelconque des revendications précédentes, où d'autres prises de vues radiographiques sont effectuées à partir d'autres positions de déplacement et sont rattachées dans une forme adaptée à la première prise de vue radiographique ou à une représentation globale générée à partir de cette dernière en correspondance avec la deuxième prise de vue radiographique.

12. Procédé selon l'une quelconque des revendications précédentes, où un premier jeu de prises de vues comportant la première et la deuxième prise de vue, est généré par rapport à une première position commune, et un deuxième jeu de prises de vues comportant une autre première et deuxième prise de vue est généré dans une deuxième position commune différente de la première, où les positions communes sont disposées à un angle l'une par rapport à l'autre et/ou situées à distance l'une de l'autre.

13. Procédé selon la revendication 12, où l'angle dépasse une valeur minimale d'au moins environ 30°, de préférence environ 60° et en particulier au moins environ 90°.

14. Procédé selon l'une quelconque des revendications précédentes, où les signaux de commande sont générés par l'appareil radiographique ou le système de navigation à partir de la résolution d'une prise de vue fournie par une analyse d'image.
